# EUROPÄISCHE PATENTSCHRIFT

(11) **EP 0 369 941 B1**
(45) Veröffentlichungstag und Bekanntmachung des Hinweises auf die Patenterteilung: **02.02.1994**
(21) Anmeldenummer: 89810837.8
(22) Anmeldetag: 06.11.1989
(51) Int. Cl.: C07D 309/38

(54) **Substituierte alfa-Pyrone und Verfahren zu deren Herstellung**
Substituted alpha-pyrones and process for their preparation
Alpha-pyrones substituées et procédé pour leur préparation

(30) Priorität: 15.11.1988 CH 4224/88
(43) Veröffentlichungstag der Anmeldung: 23.05.1990
(73) Patentinhaber: CIBA-GEIGY AG, 4002 Basel (CH)
(72) Erfinder: Vratislav, Kvita, Dr., CH-4153 Reinach (CH)

(56) Entgegenhaltungen:
- JOURNAL OF MEDICINAL CHEMISTRY, Band 29, 1986, Seiten 1159-1163, American Chemical Society, Washington, US; W.A. BOULANGER et al.: "Structure-activity study of 6-substituted 2-pyranones as inactivators of alpha-chymotrypsin"

## Beschreibung

Die Erfindung betrifft 4- oder 4,6-chlorierte oder -bromierte α-Pyrone und ein Verfahren zur Herstellung von 4-Chlor- oder 4-Brom-α-pyronen.

W.A. Boulanger et al. beschreiben in J. Med. Chem., 29, 1159-1163 (1986) die Herstellung von 4-Phenyl-6-chlor-α-pyron und dessen Reduktion mit Zink und Eisessig zum 4-Phenyl-α-pyron. A. Roedig et al. erwähnen in Liebigs. Ann. Chem., Bd. 636, 1-18 (1961), dass im Perchlor-α-pyron die beweglichen Chloratome in 4- und 6-Stellung leicht mit Zink und Essigsäure reduktiv entfernt werden können.

Dagegen wurde nun gefunden, dass die Reduktion von α-Pyronen, die in 4-und 6-Stellung mit Cl und/oder Br substituiert sind, in guten Ausbeuten und hoher Selektivität 4-Chlor- bzw. 4-Brom-α-pyron ergibt.

Ein Gegenstand der Erfindung sind α-Pyrone der Formel I 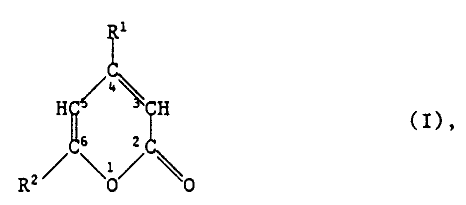
worin R¹ Cl oder Br und R² H, Cl oder Br sind.

R¹ steht bevorzugt für Cl und R² bevorzugt für H oder Cl. Besonders bevorzugte Verbindungen sind 4-Chlor-α-pyron und 4,6-Dichlor-α-pyron.

Ein weiterer Gegenstand der Erfindung ist ein Verfahren zur Herstellung von α-Pyronen der Formel Ia 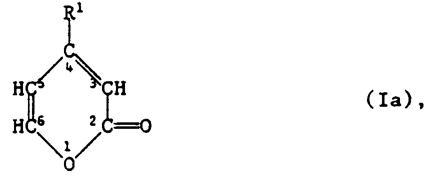
worin R¹ Cl oder Br bedeutet, dadurch gekennzeichnet, dass man ein α-Pyron der Formel Ib 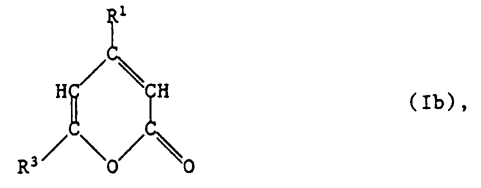
worin R¹ Cl oder Br und R³ Cl oder Br bedeuten, mit Wasserstoff reduziert.

Die Reduktion ist an sich bekannt und z.B. im J. Med. Chem., 29, 1159-1163 (1986) beschrieben. Bevorzugt wird nascierender Wasserstoff zur Reduktion verwendet, der in an sich bekannter Weise durch Einwirkung einer Säure auf ein Metall erzeugt wird, z.B. mit Zn/HCl und bevorzugt mit Zn/Essigsäure. Die Essigsäure wird bevorzugt im Ueberschuss verwendet und dient dann gleichzeitig als Lösungsmittel.

Es können aber auch inerte Lösungsmittel mitverwendet werden, z.B. aprotische und gegebenenfalls polare Lösungsmittel. Beispiele sind Kohlenwasserstoffe (Petrolether, Pentan, Hexan, Cyclohexan, Methylcyclohexan) und Ether (Diethylether, Dibutylether, Dioxan, Tetrahydrofuran, Ethylenglykoldimethylether).

Die Reaktionstemperatur kann 20 bis 100°C betragen. Das Verfahren kann so durchgeführt werden, dass man das 4,6-dihalogenierte α-Pyron in einer Säure und gegebenenfalls in einem inerten Lösungsmittel löst und das Metall portionsweise oder vollständig zugibt. Nach dem Abklingen der exothermen Reaktion kann zur Vervollständigung der Reaktion noch einige Stunden nachgerührt werden. Das Metall wird vorteilhaft in äquivalenten Mengen eingesetzt.

Zur Isolierung des gewünschten Produktes kann zunächst ein Ueberschuss der verwendeten Säure gegebenenfalls im Vakuum zusammen mit einem Lösungsmittel abdestilliert werden. Bei Mitverwendung eines Lösungsmittels kann die Reaktionsmischung direkt bzw. der in einem Lösungsmittel aufgenommene Destillationsrückstand neutralisiert werden, z.B. durch Zugabe von KHCO₃. Die Isolierung und Reinigung kann nach üblichen Verfahren wie z.B. Destillation, Umkristallisation oder chromatographische Methoden erfolgen.

Die Verbindungen der Formel Ib sind in an sich bekannter Weise durch die Cyclisierung von Glutaconsäuren der Formel II 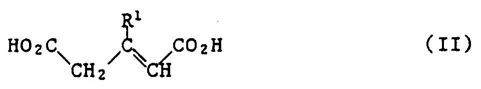
mit z.B. Acetylchlorid, Thionylchlorid, PCl₃, PCl₅ oder PBr₃ erhältlich.

Die Herstellung ist in J. Med. Chem., 29, 1159-1163 (1986) beschrieben. Die Herstellung der Glutaconsäuren der Formel II ist z.B. im J. Chem. Soc., 121, 1638 (1922) beschrieben.

In den Verbindungen der Formel Ia kann das Chlor- bzw. Bromatom überraschend mit nukleophilen Verbindungen substituiert werden, wobei die elektrophile CH-Gruppe in 6-Stellung nur in geringem Umfang angegriffen wird. Geeignete nukleophile Verbindungen sind z.B. sekundäre Amine, aliphatische und aromatische Alkohole oder Merkaptane bzw. deren Alkalimetallsalze, sowie Alkalimetallsulfinate. Geeignete Alkalimetalle sind z.B. Li, Na und K.

Die Verbindungen der Formel Ia und ihre nukleophil substituierten Derivate sind wertvolle Dienkomponenten für Diels-Alder-Reaktionen. Die Umsetzung mit 1,4-Anthrachinonen führt zu 2-substituierten Naphthacen-5,12-dionen, unter denen besonders die mit einem Merkaptan substituierten Naphthacen-5,12-dione ausgezeichnete Photoinitiatoren oder Photosensibilisatoren für ethylenisch ungesättigte photopolymerisierbare bzw. photodimerisierbare Verbindungen sind.

Die nachfolgenden Beispiele erläutern die Erfindung.

### A) Herstellungsbeispiele

### Beispiel 1: 4,6-Dichlor-2-oxo-2H-pyran (4,6-Dichlor-α-pyron)

Zu 291,15 g (1,398 mol) mit Eis gekühltem PCl₅ werden 114,93 g (0,699 mol) 3-Chlorglutaconsäure zugefügt. Es setzt eine heftige Reaktion unter starker HCl-Gasentwicklung ein, und es entsteht eine rote Lösung. Das Reaktionsgemisch wird noch 15 min bei 100°C gerührt und anschliessend das entstandene POCl₃ im Wasserstrahlvakuum bei 40°C abdestilliert. Der Rückstand wird in 2000 ml CH₂Cl₂ aufgenommen, mit Wasser extrahiert und über "Hyflo" filtriert. Die organische Lösung wird mit wässriger NaHCO₃-Lösung ausgerührt. Der pH-Wert darf dabei 7-7,5 nicht übersteigen. Die mit Na₂SO₄ getrocknete organische Phase wird bei 45°C im Wasserstrahlvakuum eingeengt und der Rückstand mit einem Wurstkolben destilliert (Bad 80-110°C/0,05 mbar). Ausbeute: 27,6 g (71,9 %); Schmelzpunkt: 43-45°C.

### Beispiel 2: 4-Chlor-2-oxo-2H-pyran (4-Chlor-α-pyron)

Zu einer Lösung von 49,5 g (0,3 mol) 4,6-Dichlor-2-oxo-2H-pyran in 150 ml Essigsäure werden in einer Portion 21,6 g (0,3 mol) Zn zugegeben. Es tritt eine exotherme Reaktion ein. Nach dem Abklingen der Reaktion wird das Reaktionsgemisch noch 2 Stunden gerührt. Die Essigsäure wird im Wasserstrahlvakuum bei 50°C abdestilliert, der Rückstand in 300 ml CH₂Cl₂ aufgenommen und mit 100 ml Wasser versetzt. Unter intensivem Rühren wird dann portionsweise KHCO₃ solange zugegeben, bis der pH-Wert 7 erreicht ist. Die organische Phase wird abgetrennt, und die wässrige Lösung wird noch mit 2 mal 100 ml CH₂Cl₂ extrahiert. Die vereinten organische Phasen werden mit Na₂SO₄ getrocknet und eingeengt. Der Destillationsrückstand wird abgesaugt und der kristalline Rückstand mit Ether nachgewaschen. Die Mutterlaugen werden über Silikagel chromatographiert (Laufmittel CH₂Cl₂). Gesamtausbeute: 12,43 g (31,7 % der Theorie); Schmelzpunkt: 56-58°C.

### B) Anwendungsbeispiel

### a) 4-Benzylthio-2-oxo-2H-pyran (4-Benzylthio-α-pyron)

In eine Lösung von 0,433 g (0,0033 mol) 4-Chlor-2-oxo-2H-pyran und 0,41 g (0,0033 mol) Benzylmerkaptan in 2 ml Dimethylsulfoxid werden 0,228 g (0,00165 mol) K₂CO₃ zugefügt. Nach einer Stunde Rühren wird noch einmal die gleiche Menge K₂CO₃ zugegeben. Das Reaktionsgemisch wird dann über Nacht bei 25°C gerührt, mit 30 ml CH₂Cl₂ verdünnt und 5 mal mit 50 ml gesättigter wässriger NaCl-Lösung extrahiert. Die organische Phase wird mit Na₂SO₄ getrocknet und im Vakuum der Wasserstrahlpumpe eingeengt. Der Destillationsrückstand wird über Silikagel chromatographiert. Ausbeute: 0,36 g (50 %); Schmelzpunkt: 105-106 °C.

### b) 2-Benzylthio-naphthacen-5,12-dion

Ein Gemisch von 0,27 g (0,0012 mol) 4-Benzylthio-2-oxo-2H-pyran, 0,25 g (0,0012 mol) 1,4-Anthrachinon und 0,10 g (0,0012 mol) MnO₂ in 5 ml Dichlorbenzol wird 24 Stunden am Rückfluss (180°C) erhitzt, abgekühlt und über Silicagel chromatographiert. Mit Cyclohexan als Laufmittel wird zuerst Dichlorbenzol extrahiert, dann wird mit CH₂Cl₂ chromatographiert. Das vorgereinigte Produkt wird nochmals chromatographiert (Laufmittel CH₂Cl₂) und das Lösungsmittel eingeengt. Ausbeute: 0,09 g (20 %); Schmelzpunkt: 200-203°C.

### c) Photohärtung eines Acrylat-Gemisches zur Herstellung eines Reliefbildes.

Es wird eine photohärtbare Zusammensetzung hergestellt durch Mischen der folgenden Komponenten:

| | Feststoffgehalt |
|---|---|
| 150,30 g Scripset 540¹⁾ (30 %-ige Lsg. in Aceton) | 45,1 g |
| 48,30 g Trimethylolpropantriacrylat | 48,3 g |
| 6,60 g Polyethylenglykoldiacrylat | 6,6 g |
| 0,08 g Kristallviolett | |
| 205,28 g | 100,0 g |

| | |
|---|---|
| ¹⁾ Polystyrol-Maleinsäurehalbester-Copolymer (Monsanto) | |

Portionen dieser Zusammensetzung werden mit 0,2 % (bezogen auf die Zusammensetzung) 2-Benzylthio-naphthacen-5,12-dion vermischt. Alle Operationen werden unter Rotlicht oder Gelblicht ausgeführt.

Die Proben werden mit einem Spiralrakel von 150 µm auf 200 µm Aluminiumfolie (10 x 15 cm) aufgetragen. Das Lösungsmittel wird durch Erwärmung auf 60°C während 15 Minuten im Umluftofen entfernt. Es resultiert eine Trockenschichtdicke von 35 µm. Auf die Schicht wird eine 76 µm dicke Polyesterfolie gelegt und auf diese ein standardisiertes Testnegativ mit 21 Stufen verschiedener optischer Dichte (Stouffer-Keil) gelegt. Darüber wird eine zweite Polyesterfolie gelegt und das so erhaltene Laminat mittels Vakuum auf einer Metallplatte fixiert. Die Probe wird dann mit einer 5 KW-Metallhalogenid-Lampe (Typ MO 23) im Abstand von 30 cm belichtet und zwar in einer ersten Testreihe 20 Sekunden und einer zweiten Testreihe 40 Sekunden. Nach der Belichtung werden die Folien und die Maske entfernt, die belichtete Schicht in einem Ultraschallbad 120 Sekunden mit Entwickler A 2 Minuten lang entwickelt und anschliessend bei 60°C 15 Minuten im Umluftofen getrocknet. Die Empfindlichkeit des verwendeten Initiatorsystems wird durch die Angabe der letzten klebefrei abgebildeten Keilstufe charakterisiert. Je höher die Zahl der Stufen ist, desto empfindlicher ist das System. Eine Erhöhung um zwei Stufen bedeutet dabei etwa eine Verdopplung der Härtungsgeschwindigkeit. Die Zahl der abgebildeten Stufen beträgt nach 20 s Belichtung 1 und nach 40 s Belichtung 4. (Entwickler A enthält 15 g Natriummetasilikat·9 H₂O; 0,16 g KOH; 3 g Polyethylenglykol 6000; 0,5 g Lävulinsäure und 1000 g deionisiertes Wasser).

## Patentansprüche

1. α-Pyrone der Formel I 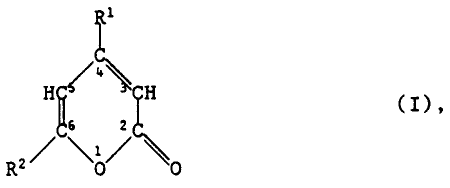 worin R¹ Cl oder Br und R² H, Cl oder Br sind.

2. α-Pyrone der Formel I gemäss Anspruch 1, dadurch gekennzeichnet, dass es sich um 4-Chlor-α-pyron handelt.

3. α-Pyrone der Formel I gemäss Anspruch 1, dadurch gekennzeichnet, dass es sich um 4,6-Dichlor-α-pyron handelt.

4. Verfahren zur Herstellung von α-Pyronen der Formel Ia 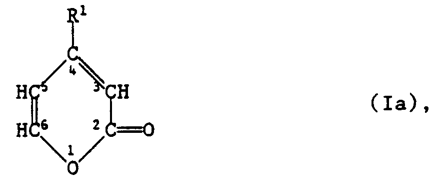 worin R¹ Cl oder Br bedeutet, dadurch gekennzeichnet, dass man ein α-Pyron der Formel Ib 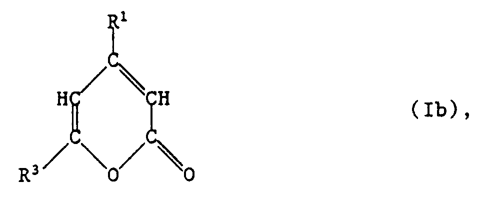 worin R¹ Cl oder Br und R³ Cl oder Br bedeuten, mit Wasserstoff reduziert.

5. Verfahren gemäss Anspruch 4, dadurch gekennzeichnet, dass man ein inertes Lösungsmittel mitverwendet.

6. Verfahren gemäss Anspruch 4, dadurch gekennzeichnet, dass man die Reduktion mit einem Gemisch aus Zn und Essigsäure durchführt.

7. Verfahren gemäss Anspruch 6, dadurch gekennzeichnet, dass die Essigsäure im Ueberschuss verwendet wird und gleichzeitig als Lösungsmittel dient.

8. Verfahren zur Herstellung von Verbindungen der Formel Ib 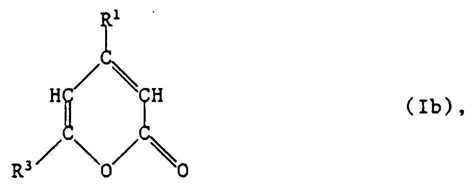 worin R¹ Cl oder Br und R³ Cl oder Br bedeuten, dadurch gekennzeichnet, dass man eine Verbindung der Formel II 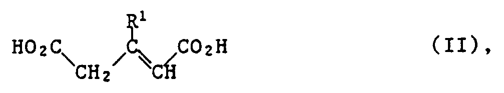 worin R¹ Cl oder Br ist, in Gegenwart von Acetylchlorid, Thionylchlorid, Phosphortrichlorid, Phosphortribromid oder Phosphorpentachlorid cyclisiert.

## Claims

1. An α-pyrone of formula I 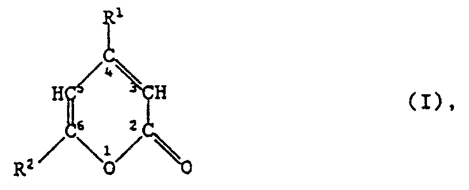 in which R¹ is chloro or bromo and R² is hydrogen, chloro or bromo.

2. An α-pyrone of formula I according to claim 1, which is 4-chloro-α-pyrone.

3. An α-pyrone of formula I according to claim 1, which is 4,6-dichloro-α-pyrone.

4. A process for the preparation of an α-pyrone of formula Ia 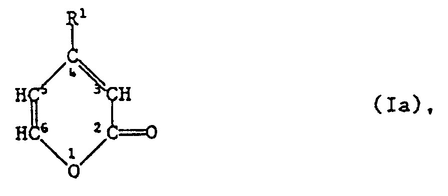 in which R¹ is chloro or bromo, which comprises reducing an α-pyrone of formula Ib 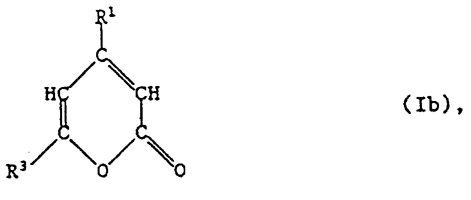 in which R¹ is chloro or bromo and R³ is chloro or bromo, with hydrogen.

5. A process according to claim 4, wherein an inert solvent is concurrently used.

6. A process according to claim 4, wherein the reaction is carried out with a mixture of zinc and acetic acid.

7. A process according to claim 6, wherein the acetic acid is used in excess and acts simultaneously as solvent.

8. A process for the preparation of a compound of formula Ib 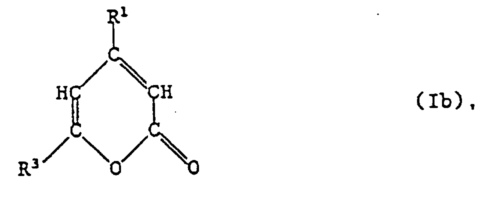 in which R¹ is chloro or bromo and R³ is chloro or bromo, which comprises cyclizing a compound of formula II 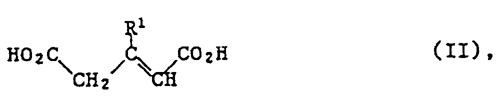 in which R¹ is chloro or bromo, in the presence of acetyl chloride, thionyl chloride, phosphorus trichloride, phosphorus tribromide or phosphorus pentachloride.

## Revendications

1. Alpha-pyrones de formule I 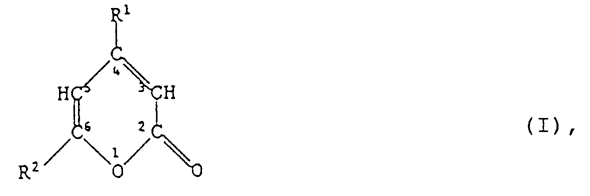 dans laquelle R¹ est Cl ou Br et R² est H, Cl ou Br.

2. Alpha-pyrones de formule I suivant la revendication 1, caractérisées en ce qu'il s'agit de la 4-chloro-alpha-pyrone.

3. Alpha-pyrones de formule I suivant la revendication 1, caractérisées en ce qu'il s'agit de la 4,6-dichloro-alpha-pyrone.

4. Procédé de préparation d'alpha-pyrones de formule Ia 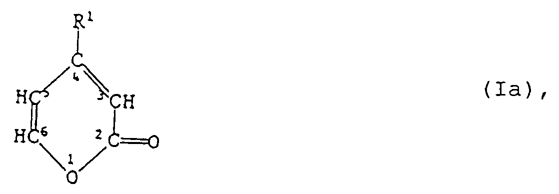 dans laquelle R¹ représente Cl ou Br, caractérisé en ce que l'on réduit avec l'hydrogène une alpha-pyrone de formule Ib 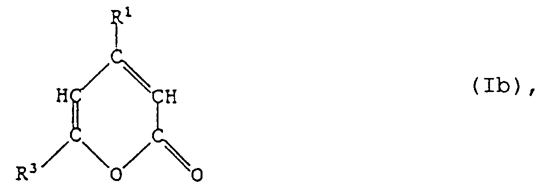 dans laquelle R¹ représente Cl ou Br et R³ représente Cl ou Br.

5. Procédé suivant la revendication 4, caractérisé en ce que l'on utilise en association un solvant inerte.

6. Procédé suivant la revendication 4, caractérisé en ce que l'on effectue la réduction avec un mélange de Zn et d'acide acétique.

7. Procédé suivant la revendication 6, caractérisé en ce que l'acide acétique est utilisé en excès et sert en même temps de solvant.

8. Procédé de fabrication de composés de formule 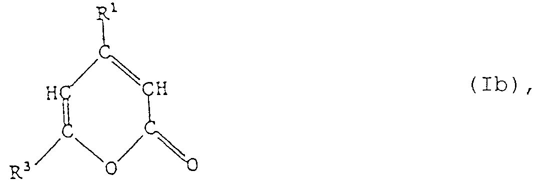 dans laquelle R¹ représente Cl ou Br et R³ représente Cl ou Br, caractérisé en ce que l'on cyclise un composé de formule II 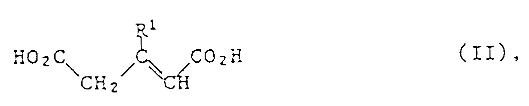 dans laquelle R¹ est Cl ou Br, en présence de chlorure d'acétyle, de chlorure de thionyle, de trichlorure de phosphore, de tribromure de phosphore ou de pentachlorure de phosphore.
